# EUROPÄISCHE PATENTSCHRIFT

(11) **EP 0 993 427 B1**
(45) Veröffentlichungstag und Bekanntmachung des Hinweises auf die Patenterteilung: **04.09.2002**
(21) Anmeldenummer: 98932116.1
(22) Anmeldetag: 04.06.1998
(51) Int. Cl.: C07C 31/133

(54) **VERFAHREN ZUR HERSTELLUNG VON 2-CYCLODODECYL-1-PROPANOL**
METHOD FOR PRODUCING 2-CYCLODODECYL-1-PROPANOL
PROCEDE POUR LA PREPARATION DE 2-CYCLODODECYL-1-PROPANOL

(30) Priorität: 17.06.1997 DE 19725533
(43) Veröffentlichungstag der Anmeldung: 19.04.2000
(73) Patentinhaber: BASF AKTIENGESELLSCHAFT, 67056 Ludwigshafen (DE)
(72) Erfinder: EBEL, Klaus, D-68623 Lampertheim (DE); PINKOS, Rolf, D-67098 Bad Dürkheim (DE)
(86) Internationale Anmeldenummer: EP9803339
(87) Internationale Veröffentlichungsnummer: WO98057914

(56) Entgegenhaltungen:
- EP-A- 0 278 384
- FR-A- 2 357 514
- H. VOGEL: "Radical Addition of Carboxylic Acids and Carboxylic Acid Derivatives to Unsaturated Compounds as a Synthetic Method " SYNTHESIS, Nr. 3, März 1970, Seiten 99-140, XP002078231 STUTTGART DE in der Anmeldung erwähnt
- HOUBEN-WEYL: "Methoden der Organischen Chemie, 4te Auflage, Band VI/1b Alkohole III" , GEORG THIEME VERLAG , STUTTGART, NEW YORK XP002078232 siehe Seite 103 - Seite 111

## Beschreibung

Die vorliegende Patentanmeldung betrifft ein vorteilhaftes technisches Verfahren zur Herstellung von 2-Cyclododecyl-1-propanol aus Cyclododecen und Propionsäure bzw. deren Derivaten.

2-Cyclododecyl-1-propanol (auch Hydroxyambran genannt) ist ein Riechstoff der Moschus-Klasse, der zunehmend an Bedeutung gewinnt(vgl. EP 278 384 B1). Das bisherige Herstellverfahren hierfür geht von Cyclododecanon und 2-Brom-propionsäure-alkylestern aus (vgl. beispielsweise Angew. Chem.108 (1996),1312-13 oder EP 278 384). Bei diesem Verfahren wird in einer Reformatsky-Reaktion mit Zink der ungesättigte 2-Cyclododecyl-propionsäureester gebildet. Es fällt Zinkbromid an, das entsorgt werden muß. Der entstandene Ester wird anschließend katalytisch hydriert bzw. gemäß EP 278 384 mit Lithiumaluminiumhydrid reduziert. Verbindungen, die über Halogenorganyle gebildet werden, enthalten im allgemeinen noch Spuren an Halogen. Da bei der Hydrierung hieraus Halogenwasserstoff gebildet wird, ist bei der katalytischen Hydrierung des Esters zum entsprechenden Alkohol mit starker Korrosion am Reaktormaterial und am Hydrierkatalysator zu rechnen. Dabei stören bekanntermaßen bereits Halogenspuren von etwa 1 ppm.

Das vorstehend beschriebene Verfahren eignet sich gut zur Herstellung von kleinen Mengen des 2-Cyclododecyl-1-propanols. Zur technischen Herstellung, d.h. zur Herstellung von 2-Cyclododecyl-1-propanol im Tonnenmaßstab ist dieses Verfahren jedoch aus den folgenden Gründen ungeeignet:
1. Sind die als Ausgangsverbindungen verwendeten 2-Brom-propionsäurealkylester - und damit die daraus erhältlichen alpha-Brom-Zink-propionsäurealkylester - recht teuer.
2. Auch die Herstellung des Cyclododecanons durch Hydrieren von Cyclododecatrien und anschließendes Oxidieren des erhaltenen Cyclododecans ist technisch recht aufwendig und daher teuer.
3. Die bereits oben beschriebene Bildung von Halogenwasserstoffen bei der Hydrierung führt zu Korrosionsproblemen für die Reaktoren und die Hydrierkatalysatoren, was die Notwendigkeit der Verwendung extrem teurer Reaktormaterialien mit sich bringt und zu kurzen Standzeiten für die Hydrierkatalysatoren führt.
4. Das bei der Umsetzung anfallende Zinkbromid führt zu enormen Abwasserproblemen bzw erfordert aufwendige Aufarbeitungsoder Entsorgungsmaßnahmen.
5. Die in EP 278 384 beschriebene Reduktion mit Lithiumaluminiumhydrid ist für eine technische Synthese prohibitiv.
   Es war daher die Aufgabe der Erfindung, ein Verfahren für die technische Herstellung von 2-Cyclododecyl-1-propanol zu entwickeln, welches die Nachteile des Verfahrens des Standes der Technik vermeidet. Das neue Verfahren sollte also von möglichst gut zugänglichen und daher billigen Ausgangsstoffen ausgehen und sollte in möglichst wenigen und technisch leicht zu realisierenden Reaktionsschritten und in guten Ausbeuten zu einem olfaktorisch ansprechenden Produkt führen, ohne Abwasserprobleme durch hohen Salzanfall hervorzurufen.

Es wurde nun überraschend gefunden, daß sich 2-Cyclododecyl-1-propanol auch in technischem Maßstab sehr vorteilhaft herstellen läßt, wenn man Cyclododecen in Gegenwart katalytischer Mengen eines Radikalinitiators mit einem Überschuß an Propionsäure oder einem Propionsäure-Derivat umsetzt, und die gebildete 2-Cyclododecyl-propionsäure bzw. die entsprechenden 2-Cyclododecyl-propionsäure-derivate katalytisch an Hydrierkatalysatoren mit Wasserstoff hydriert.

Es war überraschend, daß man das begehrte Hydroxyambran auf diese Weise in einer technisch gut durchführbaren und nur zwei Reaktionsstufen umfassenden Synthese ausgehend von dem leicht durch Trimerisieren von Butadien und anschließende Partialhydrierung herstellbaren Cyclododecens in olfaktorisch zufriedenstellender Reinheit herstellen kann.

Aus Synthesis No.3 (1970),Seiten 99-140 war zwar schon bekannt, daß man Carbonsäuren oder Carbonsäurederivate in Gegenwart von Radikalinitiatoren an Olefine anlagern kann, jedoch werden gemäß loc. cit. bei der Umsetzung der Cycloolefine Cyclopenten und Cyclohexen - im Gegensatz zu guten Ausbeuten bei der Umsetzung mit zahllosen offenkettigen Olefinen - nur Ausbeuten von 17 bzw. 10 % der Theorie erzielt, sodaß man absolut nicht erwarten konnte, daß man bei der Umsetzung mit Cyclododecen Ausbeuten erzielen könnte, die für eine technische Herstellung befriedigen.

Auch aus "Methoden der organischen Chemie" (Houben-Weyl) Band V/1b 4. Auflage 1997, Seiten 1058-1063 ist die radikalische Addition von Carbonsäuren bzw. Carbonsäurederivaten an Olefine schon bekannt. Die Addition von Propionsäure oder deren Derivaten an Cyclododecen als Olefin wurde jedoch noch nirgends erwähnt.
Da bei durch Radikalinitiatoren eingeleiteten Reaktionen im allgemeinen größere Mengen an unerwünschten Nebenprodukten gebildet werden, war zudem nicht zu erwarten, daß Hydroxyambran durch eine solche Reaktion in olfaktorisch ausreichender Reinheit hergestellt werden kann.

Gegenstand der Erfindung ist dementsprechend ein Verfahren zur Herstellung von 2-Cyclododecyl-1-propanol das dadurch gekennzeichnet ist, daß man
A. Cyclododecen in Gegenwart von katalytischen Mengen eines Radikalinitiators mit Propionsäure oder einem Propionsäure-Derivat umsetzt und
B. die gebildete 2-Cyclododecyl-propionsäure bzw. das entsprechende Derivat bei Temperaturen von 100 bis 300°C und Drucken von 20 bis 350 bar an geeigneten Hydrierkatalysatoren mit Wasserstoff umsetzt.

Mit besonderem Vorteil gelingt das erfindungsgemäße Verfahren, wenn man als Derivate der Propionsäure deren Ester mit niederen (C₁- bis C₆-) Alkanolen verwendet.

Besonders vorteilhaft gestaltet sich das erfindungsgemäße Verfahren, wenn man als Radikalinitiator Wasserstoffperoxid, Perborate, Perdisulfate, Permonosulfate, Persäuren, Hydroperoxide, Dialkylperoxide, Perester, Diacylperoxide, Peroxydicarbonate, Perketale oder Ketonperoxide, insbesondere das gut zugängliche Di-tert.butylperoxid, verwendet.

Der zweite Reaktionsschritt, die katalytische Hydrierung, gelingt gut, wenn man als Hydrierkatalysator einen Katalysator verwendet, der eins oder mehrere der Elemente der Gruppen Ib, VIb, VIIb und VIIIb, sowie IIIa, IVa und Va des Periodensystems der Elemente enthält, insbesondere, wenn man einen Hydrierkatalysator verwendet, der mindestens eines der Elemente Kupfer, Kobalt oder Rhenium enthält.

Das als Ausgangsverbindung verwendete Cyclododecen läßt sich leicht durch Trimerisierung von Butadien zu Cyclododecatrien (vgl.Angew. Chem.75 (1963), 10) und anschließende Partialhydrierung desselben herstellen. Ein vollständiges Hydrieren und das anschließende Oxidieren des erhaltenen Cyclododecans zu Cyclododecanon- wie bei dem bekannten Herstellverfahren - ist also nicht notwendig.

Die zur Addition der Carbonsäure bzw. deren Derivaten notwendigen Radikale können nach allgemein bekannten Methoden erzeugt werden. Beispielhaft seien Belichtung und Zerfall von Radikalinitiatoren wie Peroxiden genannt. Der Zerfall von Peroxiden ist bevorzugt. Als Beispiele für Peroxide seien Wasserstoffperoxid, Perborate, Perdisulfate, Permonosulfate, Persäuren, Hydroperoxide, Dialkylperoxide, Perester, Diacylperoxide, Peroxydicarbonate, Perketale und Ketonperoxide genannt. Mit Vorteil wird der Zerfall der Radikalinitiatoren thermisch induziert. Die Perverbindungen werden in katalytischen Mengen eingesetzt. So verwendet man erfindungsgemäß im allgemeinen 0,01 bis 1, vorzugsweise 0,05 bis 0,8, insbesondere 0,1 bis 0,6 Moläquivalente Perverbindung pro Mol cyclischem Olefin. Die Reaktionstemperatur bei der radikalischen Addition richtet sich nach der eingesetzten Perverbindung. Da jede Perverbindung bei unterschiedlichen Temperaturen zu zerfallen beginnt, ist der Temperaturbereich für das erfindungsgemäße Verfahren recht groß. Er liegt im allgemeinen zwischen 30 und 250°C. Wird beispielsweise Di-tert.-butylperoxid eingesetzt, so wird bevorzugt bei 120-160°C gearbeitet.

Die Reaktion wird generell bei einem Druck durchgeführt, bei dem die Reaktionspartner flüssig vorliegen, wobei dem Druck nach oben keine Grenze gesetzt ist.

Das Mol-Verhältnis von Propionsäure bzw. dessen Derivaten zu Cyclododecen liegt im allgemeinen zwischen 400 und 1, vorzugsweise zwischen 150 und 1 und insbesondere zwischen 100 und 1. Dabei dient die Propionsäure bzw., deren Derivat vorteilhaft als Lösungsmittel.

Als Derivate der Propionsäure sind für die erfindungsgemäße Umsetzung insbesondere deren Ester geeignet. Als besonders geeignete Ester seien der Methyl-, Ethyl-, Propyl-, n-Butyl-, iso-Butyl-, tert.-Butyl-, tert.-Amyl-, n-Pentyl und n-Hexylester genannt. Es können aber auch Amide oder Propionsäureanhydrid eingesetzt werden.

Die Reaktion kann absatzweise, halbkontinuierlich oder kontinuierlich durchgeführt werden.

Der Reaktionsaustrag kann vor dem zweiten Verfahrensschritt, der Hydrierung, aufgearbeitet werden, er kann aber auch direkt in die Folgestufe eingesetzt werden. Die Aufarbeitung beschränkt sich im allgemeinen auf die Abtrennung der überschüssigen Propionsäure bzw. dem überschüssigen Propionsäurederivat sowie von unumgesetztem cyclischem Olefin. Vorzugsweise werden die genannten Leichtsieder destillativ abgetrennt. Sie können in die erste Verfahrensstufe rückgeführt werden.

Will man die 2-Cyclododecyl-propionsäure rein gewinnen, so kann nach Abtrennung der Propionsäure der Rückstand selbst destilliert werden, oder die Säure durch Kristallisation in reiner Form erhalten werden. Die Hydrierung der 2-Cyclododecyl-propionsäure bzw. die Hydrierung von deren Derivate kann mit dem Rohaustrag der ersten Verfahrensstufe, dem um Propionsäure bzw. Propionsäure-Derivaten abgereicherten Rohaustrag oder mit den reinen Stoffen in der Gas- oder Flüssigphase erfolgen.

Als Hydrierkatalysatoren verwendet man im erfindungsgemäßen Verfahren im allgemeinen heterogene Katalysatoren, man kann aber auch zur Hydrierung von Carbonylgruppen geeignete homogene Katalysatoren verwenden. Sie können sowohl als Festbettkatalysatoren angeordnet werden, als auch in mobiler Form, beispielsweise in einem Wirbelbettreaktor, eingesetzt werden. Beispiele für solche Hydrierkatalysatoren hierfür sind z.B. in Houben-Weyl, Methoden der Organischen Chemie, Band IV/1c, S. 16 bis 26 beschrieben.

Von diesen Hydrierkatalysatoren werden solche bevorzugt, die ein oder mehrere Elemente der Gruppe Ib, VIb, VIIb und VIIIb, sowie IIIa, IVa und Va des Periodensystems der Elemente, insbesondere Kupfer, Chrom, Rhenium, Kobalt, Rhodium, Nickel, Palladium, Eisen, Platin, Indium, Zinn und Antimon enthalten.

Die im erfindungsgemäßen Verfahren eingesetzten Katalysatoren können z.B. sogenannte Fällungskatalysatoren sein. Solche Katalysatoren können dadurch hergestellt werden, daß man ihre katalytisch aktiven Komponenten aus deren Salzlösungen, insbesondere aus den Lösungen von deren Nitraten und/oder Acetaten, beispielsweise durch Zugabe von Alkalimetall- und/oder Erdalkalimetallhydroxid- und/oder Alkali- und/oder Erdalkali-carbonat-Lösungen, in Form ihrer schwerlöslichen Hydroxyde, Oxydhydrate, basischen Salze oder Carbonate ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischtvalentigen Oxide umwandelt, welche durch eine Behandlung mit Wasserstoff oder mit Wasserstoff enthaltenden Gasen bei in der Regel 50 - 700°C, insbesondere 100 bis 400°C zu den betreffenden Metallen und/oder oxidischen Verbindungen niederer Oxidationsstufe reduziert und dadurch in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Als Trägermaterial für die Katalysatoren des erfindungsgemäßen Verfahrens eignen sich im allgemeinen solche Trägermaterialien, bei denen die katalytisch-hydrierend wirkende Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte diese Trägerkatalysatoren an katalytisch aktiven Metallen zu höheren Raum-Zeit-Umsätzen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-% bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirken muß. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 147 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch zu Beginn der Reaktion in situ durch den anwesenden Wasserstoff erfolgen, vorzugsweise werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Tonerden, wie Montmorillonite, Silikate, wie Magnesium- oder Aluminiumsilikate, Zeolithe, wie ZSM-5 oder ZSM-10-Zeolithe, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren anwendbaren Katalysatoren dienen.

Als für das erfindungsgemäße Verfahren einsetzbare Heterogenkatalysatoren seien die folgenden beispielhaft genannt:

Kobalt auf Aktivkohle, Kobalt auf Siliciumdioxid, Kobalt auf Aluminiumoxid, Rhenium auf Aktivkohle, Rhenium auf Siliciumdioxid, Rhenium/Zinn auf Aktivkohle, Rhenium/Platin auf Aktivkohle, Kupfer auf Aktivkohle, Kupfer/Siliciumdioxid, Kupfer/Aluminiumoxid, Kupferchromit, Bariumkupferchromit, Kupfer/Aluminiumoxid/Manganoxid, Kupfer/Aluminiumoxid/Zinkoxid sowie die Katalysatoren gemäß DE-A 3 932 332, US-A 3 449 445, EP-A 44 444, EP-A 147 219, DE-A 3 904 083, DE-A 2 321 101, EP-A 415 202, DE-A 2 366 264, EP 0 552 463 und EP-A 100 406.

Besonders bevorzugte Katalysatoren enthalten mindestens eines der Metalle Kupfer, Kobalt oder Rhenium.

Das erfindungsgemäße Verfahren kann vorteilhaft kontinuierlich ausgeübt werden. Dabei können beispielsweise Rohrreaktoren eingesetzt werden, in denen der Katalysator vorteilhaft in Form eines Festbetts angeordnet ist, oder Wirbelschichtreaktoren, in denen der Katalysator durch den Gasstrom bewegt wird.

Man kann die Hydrierung mit und ohne Lösungsmittel betreiben. Wird ein Lösungsmittel eingesetzt, so kann es Wasser oder ein Alkohol wie z.B. Methanol oder Ethanol sein. Ferner eignen sich Ether wie Tetrahydrofuran, Dioxan oder Ethylenglycoldimethylether, Kohlenwasserstoffe, wie Hexan oder Cyclohexan.

Die Hydrierung wird bei Temperaturen zwischen 100 und 300°C, vorzugsweise 150 und 260°C ausgeführt. Wird die Hydrierung in der Gasphase vorgenommen, so liegt der Reaktionsdruck zwischen 20 und 80 bar. Wird sie in Flüssigphase durchgeführt,liegt der Druck zwischen 50 und 350 bar, vorzugsweise zwischen 100 und 320 bar, insbesondere zwischen 130 und 300 bar.

Das erfindungsgemäße Verfahren soll durch das nachfolgende Beispiel näher erläutert, jedoch in keiner Weise eingeschränkt werden.

### Beispiel 1

370 g (5 mol) Propionsäure, wurden in einem Reaktionsgefäß vorgelegt und auf 140°C aufgeheizt. Danach wurde ein Gemisch aus 84 g (0,5 mol) Cyclododecen und 14,6 g (0,1mol) Di-tert.-butylperoxid innerhalb von 5 Stunden (h) zugetropft. Anschließend wurde noch weitere 2 h unter Rückfluß zum Sieden erhitzt. Nach Abkühlen des Reaktionsansatzes wurde die überschüssige Propionsäure sowie der gebildete tert.-Butylalkohol bei 30 mbar abdestilliert. Nach Erniedrigung des Destillationsdrucks wurde noch nicht umgesetztes Cyclododecen abdestilliert. Es verblieben 31 g Rückstand.

Der erhaltene Rückstand wurde in 280 ml Ethanol gelöst, und in einem Autoklaven diskontinuierlich an 31 g eines KupferKatalysators, welcher in seiner unaktivierten Form folgende Zusammensetzung aufwies:
70 Massen-% CuO, 25 Massen-% ZnO und 5 Massen-% Al₂O₃, und bei 180°C mit Wasserstoff aktiviert worden war, bei 220°C und 220 bar 2 h mit Wasserstoff hydriert. Danach wurde abgekühlt und entspannt. Im Austrag fanden sich nach gaschromatographischer Analytik (GC) unter Herausrechnen von Ethanol 57,7 % 2-Cyclododecyl-1-propanol und 14 % 2-Cyclododecyl-propionsäureethylester, der Rest waren vorwiegend hochsiedende Produkte. Die Ausbeute an dem gewünschten Hydroxyambran betrug ca. 60 % der Theorie, bezogen auf umgesetztes Cyclododecen bei einem Umsatz von 64 %.

### Beispiel 2

880 g (10 mol) Propionsäuremethylester, 163 g (0,94 mol) eines 96 gew.-%igen Cyclododecens und 26 g (0,18 mol) Di-tert.-butylperoxid wurden unter Rühren 20 h bei 140°C und einem Eigendruck von 5,1 bar in einem Autoklaven umgesetzt.

Der Reaktionsaustrag enthielt nach GC-Analyse 98 g Cyclododecen und 50 g 2-Cyclododecyl-propionsäuremethylester. Das entspricht einer Selektivität von 52 % bei einem Umsatz von 60 %.

Nach Destillation wies der 2-Cyclododecyl-propionsäuremethylester einen Siedepunkt von 112°C/3 mbar auf.

### Beispiel 3

500 g eines analog Beispiel 1 durch Anlagerung von Propionsäure an Cyclododecen erhaltenen Rückstandes wurden in 500 g n-Butanol gelöst und an 25 ml eines analog Beispiel 1 von DE 23 21 101 hergestellten Kobaltkatalysators in einem Rohrreaktor in Rieselfahrweise kontinuierlich (Zulauf ca. 20 g/h, 220 bar, 220-230°C) hydriert. Zur Vervollständigung des Umsatzes wurde der Hydrieraustrag unter gleichen Bedingungen nachhydriert.

Im Austrag befanden sich - das Lösungsmittel n-Butanol herausgerechnet - ca. 70 % 2-Cyclododecyl-propanol. Die weiteren Bestandteile waren überwiegend Dimere und Oligomere des Cyclododecans. Der Hydrieraustrag wurde nach Abtrennung des Butanols in einer 1 m hohen Füllkörperkolonne bei 1 mbar fraktioniert destilliert. Bei einer Sumpftemperatur von 185°C wurden bei Siedetemperaturen zwischen 123 und 132°C insgesamt 260 g 2-Cyclododecyl-propanol mit einer Reinheit von 97-99,8 % erhalten. Alle erhaltenen Fraktionen wiesen eine einwandfreie Geruchsqualität auf.

## Patentansprüche

1. Verfahren zur Herstellung von 2-Cyclododecyl-1-propanol **dadurch gekennzeichnet, daß** man
A. Cyclododecen in Gegenwart von katalytischen Mengen eines Radikalinitiators mit Propionsäure oder einem ihrer Derivate umsetzt und
B. die gebildete 2-Cyclododecyl-propionsäure oder das entsprechende Derivat bei Temperaturen von 100 bis 300°C und Drucken von 20 bis 350 bar an geeigneten Hydrierkatalysatoren mit Wasserstoff umsetzt.

2. Verfahren gemäß Anspruch 1, **dadurch gekennzeichnet,daß** man als Derivate der Propionsäure deren Ester mit niederen Alkanolen verwendet.

3. Verfahren gemäß Anspruch 1, **dadurch gekennzeichnet, daß** man als Radikalinitiatoren Wasserstoffperoxid, Perborate, Perdisulfate, Permonosulfate, Persäuren, Hydroperoxide, Dialkylperoxide, Perester, Diacylperoxide, Peroxydicarbonate, Perketale oder Ketonperoxide verwendet.

4. Verfahren gemäß Anspruch 3, **dadurch gekennzeichnet, daß** man als Radikalinitiator Di-tert.-butylperoxid verwendet.

5. Verfahren gemäß Anspruch 1, **dadurch gekennzeichnet, daß** man als Hydrierkatalysator einen Katalysator verwendet, der eins oder mehrere Elemente der Gruppen Ib, VIb, VIIb und VIIIb sowie IIIa, IVa und Va des Periodensystems der Elemente enthält.

6. Verfahren gemäß Anspruch 1, **dadurch gekennzeichnet, daß** man einen Hydrierkatalysator verwendet, der mindestens eines der Elemente Kupfer, Kobalt oder Rhenium enthält.

7. Verfahren gemäß Anspruch 1, **dadurch gekennzeichnet, daß** man als Derivat der Propionsäure Propionsäureanhydrid verwendet.

## Claims

1. A process for preparing 2-cyclododecyl-1-propanol which comprises
A. reacting cyclododecene with propionic acid or one of its derivatives in the presence of catalytic amounts of a free-radical initiator and
B. reacting the 2-cyclododecylpropionic acid which is formed, or the corresponding derivative, with hydrogen on suitable hydrogenation catalysts at from 100 to 300°C and under from 20 to 350 bar.

2. A process as claimed in claim 1, wherein the propionic acid derivatives used are its esters with lower alkanols.

3. A process as claimed in claim 1, wherein the free-radical initiators used are hydrogen peroxide, perborates, perdisulfates, permonosulfates, peracids, hydroperoxides, dialkyl peroxides, peresters, diacyl peroxides, peroxydicarbonates, perketals or ketone peroxides.

4. A process as claimed in claim 3, wherein di-tert-butyl peroxide is used as free-radical initiator.

5. A process as claimed in claim 1, wherein the hydrogenation catalyst used comprises one or more elements of groups Ib, VIb, VIIb and VIIIb, and IIIa, IVa and Va, of the Periodic Table of the Elements.

6. A process as claimed in claim 1, wherein the hydrogenation catalyst used comprises at least one of the elements copper, cobalt or rhenium.

7. A process as claimed in claim 1, wherein propionic anhydride is used as propionic acid derivative.

## Revendications

1. Procédé pour la préparation du 2-cyclododécyl-1-propanol, **caractérisé par le fait que**
A. on fait réagir le cyclododécène en présence de quantités catalytiques d'un inducteur radicalaire avec l'acide propionique ou l'un de ses dérivés et
B. on fait réagir l'acide 2-cyclododécyl-propionique formé ou le dérivé correspondant avec de l'hydrogène sur des catalyseurs d'hydrogénation appropriés à des températures de 100 à 300°C et des pressions de 20 à 350 bar.

2. Procédé selon la revendication 1, **caractérisé par le fait que** l'on utilise en tant que dérivés de l'acide propionique ses esters d'alcanols inférieurs.

3. Procédé selon la revendication 1, **caractérisé par le fait que** l'on utilise en tant qu'inducteurs radicalaires le peroxyde d'hydrogène, des perborates, des perdisulfates, des permonosulfates, des peracides, des hydroperoxydes, des peroxydes de dialkyle, des peresters, des peroxydes de diacyle, des peroxydicarbonates, des peracétals ou des peroxydes de cétones.

4. Procédé selon la revendication 3, **caractérisé par le fait que** l'inducteur radicalaire utilisé est le peroxyde de di-tert-butyle.

5. Procédé selon la revendication 1, **caractérisé par le fait que** le catalyseur d'hydrogénation utilisé est un catalyseur contenant un ou plusieurs éléments des groupes Ib, VIb, VIIb et VIIIb et des groupes IIIa, IVa et Va de la Classification Périodique des Eléments.

6. Procédé selon la revendication 1, **caractérisé par le fait que** l'on utilise un catalyseur d'hydrogénation qui contient au moins un des éléments cuivre, cobalt ou rhénium.

7. Procédé selon la revendication 1, **caractérisé par le fait que** l'on utilise en tant que dérivé de l'acide propionique l'anhydride propionique.
